# EUROPÄISCHE PATENTSCHRIFT

(11) **EP 1 750 735 B1**
(45) Veröffentlichungstag und Bekanntmachung des Hinweises auf die Patenterteilung: **13.02.2008**
(21) Anmeldenummer: 04804098.4
(22) Anmeldetag: 20.12.2004
(51) Int. Cl.: A61K 36/85, A61P 1/00, A61P 19/02

(54) **VERWENDUNG EINES EXTRAKTS VON ALOYSIA/VERBENA/LIPPIA TRIPHYLLA/CITRIODORA ZUR HERSTELLUNG EINES MEDIKAMENTS ZUR BEHANDLUNG CHRONISCHER UND/ODER ENTZÜNDLICHER ERKRANKUNGEN**
USE OF AN EXTRACT OF ALOYSIA/VERBRENA/LIPPIA TRIPHYLLA/CITRIDORA FOR THE PREPARATION OF A MEDICAMENT FOR THE TREATMENT OF CHRONIC AND/OR INFLAMMATORY DISEASES
UTILISATION D'UN EXTRAIT D'ALOYSIA/VERBENA/LIPPIA TRIPHYLLA/CITRIODORA POUR LA PRÉPARATION D'UN MÉDICAMENT DESTINÉ AU TRAITEMENT DES AFFECTIONS CHRONIQUES ET/OU INFLAMMATOIRES

(30) Priorität: 18.12.2003 DE 10359384
(43) Veröffentlichungstag der Anmeldung: 14.02.2007
(73) Patentinhaber: Anoxymer GmbH, 73728 Esslingen a. N. (DE)
(72) Erfinder: BALAN, Karim, 93055 Regensburg (DE); PAPER, Dietrich, 93142 Maxhütte-Haidhof (DE)
(74) Vertreter: Behnisch, Werner
(86) Internationale Anmeldenummer: PCT/EP2004/014499
(87) Internationale Veröffentlichungsnummer: WO 2005/058338

(56) Entgegenhaltungen:
- DE-A- 10 047 835
- US-A- 4 859 468
- ANOXYMER: "Planox L" INTERNET ARTIKEL, [Online] 15. Dezember 2003 (2003-12-15), XP002325344 Gefunden im Internet: URL:http://www.anoxymer.de/b/content.php?i d=46> [gefunden am 2005-04-15]
- KIM, S. ET AL.: "Cytotoxicity of Methanol Extracts of Edible Herbs Against L1210 Cells with the Changes of Antioxidant Enzymes Activities" KOREAN JOURNAL OF PHARMACOGNOSY, Bd. 33, Nr. 4, 2002, Seiten 376-383, XP008045829
- MATSUNAGA, K. ET AL.: "Inhibitory Action of Paraguayan Medicinal Plants on 5-Lipoxygenase" NATURAL MEDICINES, Bd. 54, Nr. 3, Juni 2000 (2000-06), Seiten 151-154, XP008045847
- LAPORTA, O. ET AL.: "Bifunctional antioxidative oligosaccharides with antiinflammatory activity for joint health" AGRO FOOD INDUSTRY HI-TECH, Bd. 15, Nr. 5, Oktober 2004 (2004-10), Seiten 30-33, XP008045822

## Beschreibung

Die Erfindung betrifft die Verwendung eines Extraktes von Aloysia triphylla (L'Her.) O. Kuntze/Britt. (syn. Lippia citriodora H. B. K., Lippia triphylla (L"Her.) O. Kuntze) oder eines Lyophilisates davon, zur Chemoprävention und zur Behandlung von chronischen Erkrankungen. Die vorliegende Erfindung betrifft weiterhin einen aus Aloysia triphylla hergestellten Extrakt.

Für Aloysia triphylla existieren in der Literatur verschiedene synonyme Begriffe: Aloysia triphylla *(L'Hérit.) Britt.* / Lippia citriodora *H.B.K.* / Verbena triphylla *L'Herit.,* Zitronenkraut, Verbenenkraut u.v.a.

Für Extrakte von Aloysia triphylla (Verbenenkraut) fehlen neuere pharmakologische Untersuchungen. Die bekannten Untersuchungen befassen sich in der Regel mit dem ätherischen Öl. Die Blätter der kleinen Sträucher, die man auch Verbenen nennt, enthalten große Mengen dieses ätherischen Öls, dessen Geruch für eine weite Verbreitung als Ziergewächs gesorgt hat.

Die Droge bzw. Extrakte werden in Frankreich traditionell zur symptomatischen Behandlung von Verdauungsbeschwerden einerseits und Nervosität und Schlafstörungen andererseits von den Zulassungsbehörden akzeptiert.

Aloysia triphylla findet bisher in der Lebensmittelindustrie in Form von Genußtees Einsatz. In der Kosmetikindustrie werden die aus der Pflanze gewonnenen ätherischen Öle zur Parfümierung verwendet. Der Einsatz von wässrigen oder ethanolisch-wässrigen (hydrophilen) Extrakten ist nicht bekannt.

Ein Internetartikel "Planox L" vom 15.12.2003 (www.anoxymer.de) beschreibt eine pharmazeutische Zusammensetzung (Planox L), die einen wässrigen Extrakt von Aloysia triphylla enthält. Es wird jedoch kein Extrakt von Aloysia triphylla offenbart, der durch ein wässrig-alkoholisches Lösungsmittel gewonnen wird und zusätzlich in Wasser lösbar ist.

US-A-4 859 468 offenbart in Spalte 5, Zeilen 4 ff., daß Blätter von Lippia citriodora einer wäßrig-ethanolischen Extraktion und Filtration unterworfen und dann gefriergetrocknet werden. So wird die Herstellung eines Lyophilsates aus Lippia citriodora erwähnt, wobei ein Teil Droge mit 100 Teilen heißem Wasser extrahiert wird. Die Lösung wird sodann filtriert und gefriergetrocknet. Ein wie oben geschilderter Extrakt lässt sich hieraus jedoch nicht ableiten.

In DE 100 47 835 wird ein Extrakt beschrieben, der auf Phenylethanoide bzw. Flavonoide standardisiert ist. Dieser Extrakt hemmt die physiologische und pathologische Angiogenese. Der Extrakt ist aufgrund von fettlöslichen Bestandteilen z.B. Chlorophyll nicht komplett wasserlöslich. Das heißt, der in der vorliegenden Anmeldung beschriebene Extrakt hat andere chemisch-physikalische und pharmakologische Eigenschaften. Darüber hinaus enthält DE 100 47 835 keine Information darüber, daß der Extrakt in Wasser lösbar ist.

Überraschenderweise ist nunmehr festgestellt worden, dass Extrakte von Aloysia triphylla, insbesondere Lyophilisate davon, unerwartete neue pharmakologische Wirkungen zeigen, die weder vorbeschrieben noch durch die bekannten Inhaltsstoffe und bisherigen Indikationen der Droge nahegelegt sind.

Es ist deswegen die Aufgabe der vorliegenden Erfindung, einen neuen und breit anwendbaren Extrakt von Aloysia triphylla bereitzustellen.

Diese Aufgabe wird durch den Gegenstand der unabhängigen Ansprüche gelöst. Bevorzugte Ausführungsformen sind in den abhängigen Ansprüchen angegeben.

Die Erfinder haben überraschenderweise herausgefunden, dass ein neuer, auf Basis hydrophiler Lösungsmittel aus Aloysia triphylla gewonnener Extrakt, eine breite Vielzahl therapeutischer Anwendungen aufweist, die weder der Pflanze in ihrer Gesamtheit noch Fraktionen hiervon bislang zugeschrieben wurden.

Die Erfindung betrifft so die Verwendung eines Extraktes von Aloysia triphylla, vorzugsweise des Trockenextraktes, der Urtinktur, des Fluidextraktes bzw. eines Lyophilisates davon, zur Behandlung und Prävention von rheumatoider Arthritis und anderen chronischen Erkrankungen.

Das Hauptmerkmal des erfindungsgemäßen Extraktes von Aloysia triphylla besteht darin, dass der Extrakt durch ein oder mehrere hydrophile Lösungsmittel aus dem pflanzlichen Ausgangsmaterial gewonnen wird und in Wasser im wesentlichen vollständig lösbar ist.

Der Extrakt zeigt unter anderem eine ausgezeichnete entzündungshemmende Wirkung und kann daher in zahlreichen Gebieten Anwendung finden, z.B. der Medizin, der Kosmetik etc.

Wie bereits oben angesprochen kann der Extrakt in vielen, bereits bekannten Formen vorliegen, z.B. als Trockenextrakt, Urtinktur, Fluidextrakt, Spezialextrakt oder deren Lyophilisate. Auch umfasst der Begriff "Extrakt" wie hierin verwendet Fraktionen, d.h. wirkstoffhaltige Untergruppen des Extraktes, die z.B. durch weitere Behandlung mit einzelnen hydrophilen Lösungsmitteln gewonnen wurden.

Die Herstellung eines Spezialextraktes von Aloysia triphylla mit hohem Gehalt an Wirkstoffen wird beispielsweise nach Extraktion mit Lösungsmitteln auf Wasser/Alkoholbasis, anschließendem Ausschütteln durch organische Lösungsmittel wie z.B. Aceton, Chloroform, Dichlormethan, Ethylacetat etc. und anschließender chromatographischer Reinigung z.B. an Kieselgel oder RP18-Material erreicht.

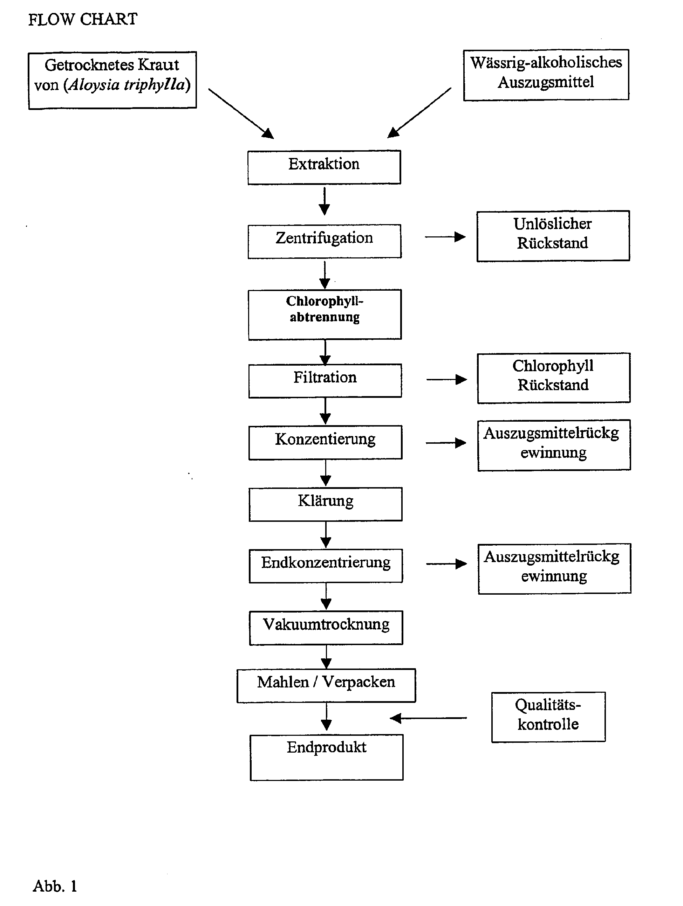

Die Herstellung eines komplett wasserlöslichen Spezialextraktes aus Aloysia triphylla ist in Abb. 1 dargestellt. Der Herstellungsprozess ist auch in den Beispielen wiedergegeben.

Fluidextrakte und Spezialextrakte (komplett wasserlösliche Extrakte, eingestellt auf einen Mindestgehalt an antioxidativen Oligosacchariden) und deren Herstellung sind im Stand der Technik bekannt. Der Fachmann auf dem Gebiet ist jederzeit dazu in der Lage die Herstellungsbedingungen zu variieren, um zu anwendbaren Zusammensetzungen zu gelangen. Einschlägige Vorschriften sind insbesondere im DAB 2004 oder EAB 4. Ausgabe, 7. Nachtrag zu finden. Weitere einschlägige Fachinformationen sind in Fachbüchern der pharmazeutischen Technologie enthalten, z.B. *"Pharmazeutische Technologie",* Rudolf Voigt, 9., völlig überarb. Auflage, oder *"Remington's Pharmaceutical Sciences",* Mack Publishing Co., Easton, PA, 18. Ausgabe.

Bei den hier beschriebenen Aloysiaextrakten und deren Fraktionen handelt es sich um Extrakte, die durch wässrig-ethanolische Extraktion aus den oberirdischen Pflanzenteilen von Aloysia triphylla gewonnen werden und danach im wesentlichen vollständig in Wasser lösbar sind. Diese Präparationen zeichnen sich durch einen bestimmten Gehalt an nicht flüchtigen, hydrophilen antioxidativen Oligosacchariden aus. Dies ist ein wesentlicher Unterschied zu den ansonsten bisher im Markt verwendeten Aloysiaextrakten, welche durch Wasserdampfdestillation gewonnen werden und die flüchtige, lipophile ätherische Öle enthalten.

Der Begriff "antioxidative Oligosaccharide" wie hierin verwendet bedeutet Oligosaccharide, die z.B. Di-, Tri-, bis Nonasaccharide sind und die mit antioxidativen Gruppen, z.B. Kaffeesäure(n), 3,4-Dihydrophenylethanol, Luteolin etc. substituiert sind. Es sei an dieser Stelle erwähnt, dass nach umfangreichen Forschungarbeiten durch die Erfinder festgestellt wurde, dass diese antioxidativen Gruppen alleine nicht die Wirkung des Gesamtextraktes zeigen. Vielmehr ist es die Mischung der in den erfindungesgemäßen Extrakten vorliegenden antioxidativen Oligosaccharide, die eine optimale Wirkung entfalten.

Die genau verwendeten Ethanol-Wassermischungen, die Extraktionstemperatur, die Extraktionsdauer und die Menge an Auszugsmittel variieren von Charge zu Charge des pflanzlichen Ausgangsmaterials und sind abhängig vom Gehalt der antioxidativen Oligosaccharide und dem Gehalt an unerwünschten Begleitstoffen (wie z.B. Chlorophyll, Carotinoide und andere insbesondere lipophile Bestandteile). Somit muß das Verfahren zur Gewinnung der erfindungsgemäßen Extrakte abhängig von der Art des verwendeten pflanzlichen Ausgangsmaterials unter Umständen angepasst werden, was jedoch für den auf dem Gebiet der Phytopharmazie tätigen Fachmann kein Problem darstellen wird.

Zwei wichtige, für das Herstellungsverfahren zu berücksichtigende Kriterien sind Folgende:
a) im Extraktionsverfahren dürfen keine Temperaturen von größer 90 °C angewendet werden, das dies zur Hydrolyse der Wirkstoffe und damit zu einer Verminderung oder gar völligen Beseitigung der Wirkung des Extraktes führen würde.
b) die gewonnen Extrakte müssen im wesentlichen rückstandsfrei in Wasser lösbar sein.

Falls im Einzelfall die Frage entsteht, ob ein Extrakt die erwarteten (optimalen) Wirkungen aufweist oder nicht, kann der Aloysiaextrakt mit Hilfe des HET-CAM-Assays (unten beschrieben) auf seine Wirkung überprüft und gegebenenfalls eingestellt werden (biologische Standardisierung).

Die vorliegende Erfindung betrifft insbesondere die folgenden Aspekte und Ausführungsformen:

Gemäß einem ersten Aspekt betrifft die Erfindung einen Extrakt von Aloysia triphylla wobei der Extrakt durch ein oder mehrere hydrophile Lösungsmittel gewonnen wird und in Wasser im wesentlichen vollständig lösbar ist. In der vorliegenden Erfindung ist am meisten bevorzugt, dass der Extrakt rückstandsfrei in Wasser lösbar ist, dies kann jedoch im ein oder anderen Fall aufgrund des gewählten Extraktionsverfahrens möglicherweise nicht ganz erreicht werden. Selbstverständlich liegen auch solche Extrakte noch im Umfang der vorliegenden Erfindung.

Das zur Extraktion eingesetzte hydrophile Lösungsmittel ist vorzugsweise Wasser und Ethanol.

Gemäß einer bevorzugten Ausführungsform ist der Extrakt ein Trockenextrakt, Fluidextrakt oder ein Spezialextrakt.

Die Erfindung betrifft ebenfalls einen Extrakt, der nach dem Herstellungsverfahren nach Abbildung 1 herstellbar ist.

Der erfindungsgemäße Extrakt kann auch in lyophilisierter Form vorliegen, um die Lagerung und nachfolgende Auflösung in Wasser (z.B. Aqua ad Injectabilia) so effizient wie möglich zu gestalten.

Die Erfindung betrifft in einem zweiten Aspekt Lebensmittel, Nutraceuticals oder Kosmetika, die einen Extrakt von Aloysia triphylla wie oben definiert enthalten. Einige Beispiele für die Anwendung des Extraktes im Lebensmittelbereich finden sich in Beispiel 4.

Gemäß eines dritten Aspektes betrifft die vorliegende Erfindung eine pharmazeutische Zusammensetzung, die einen Extrakt von Aloysia triphylla wie oben definiert und einen oder mehrere pharmazeutische Hilfsstoffe/Trägerstoffe enthält.

Diese pharmazeutische Zusammensetzung ist vorzugsweise zur Verabreichung durch Injektion, zur systemischen und/oder topischen Verabreichung vorgesehen.

Gemäß eines vierten Aspektes betrifft die vorliegende Erfindung die Verwendung eines Extraktes von Aloysia triphylla bzw. eines Lyophilisates davon, bzw. eines Aloysia triphylla Extraktes eingestellt auf z.B. Flavonoide bzw. antioxidative Eigenschaften (z.B. Troloxäquivalente) zur Herstellung eines Medikamentes zur Chemoprävention und Behandlung von Colitis und chronischen Erkrankungen wie rheumatoide Arthritis, chronischen Darmerkrankungen (z.B. Morbus Crohn).

Wie oben angesprochen, werden die erfindungsgemäßen Extrakte aus der Ganzdroge ("Herba") gewonnen, d.h. aus allen oberirdischen Pflanzenteilen (Stengel, Blätter, Blüten). Es können daher auch ätherische Öle in den erfindungsgemäßen Zusammensetzungen enthalten sein, sie stellen jedoch nicht den Hauptanteil der Wirkstoffe dar.

Weiterhin betrifft die vorliegende Erfindung die Verwendung eines Extraktes von Aloysia triphylla bzw. eines Lyophilisates davon, bzw. eines Aloysia triphylla Extraktes eingestellt auf z.B. Flavonoide bzw. antioxidative Eigenschaften (z.B. Troloxäquivalente) zum Schutz des Abbaus von Knorpel in z.B. Gelenken.

Erfindungsgemäß liegt der Extrakt vorzugsweise als Fluidextrakt vor. Wie oben angesprochen, liegt die Dosierung der Fluidextrakt-Trockensubstanz zwischen 20 mg und 2 g pro Tag.

Die erfindungsgemäßen Extrakte etc. finden als Botanical, insbesondere als Arzneimittel, Nutraceutical, Functional Food, Novel Food, Bestandteil in Kosmetika (z.B. in Sonnenschutzcremes, Anti-ageingcremes und -salben, Rasierwasser, Haarpflegemitteln etc.) und Lebensmitteln, mit antioxidativen Eigenschaften, Anwendung. Mit anderen Worten ist die vorliegende Erfindung nicht auf die Verwendung der bereits bekannten und auch häufig verwendeten ätherischen Öle gerichtet.

Die Erfindung betrifft weiterhin ein Lyophilisat, das aus einem Extrakt von Aloysia triphylla wie oben definiert hergestellt worden ist, sowie ein Botanical, Nutraceutical oder Kosmetikum, das einen Extrakt von Aloysia triphylla enthält.

Zuletzt betrifft die Erfindung ein Verfahren zur Herstellung der erfindungsgemäßen Extrakte.

Erfindungsgemäß werden als Darreichungsformen bevorzugt Dragees, Hartgelatinekapseln, flüssige Zubereitungen der Fluidextrakt-Trockensubstanz als Oralia, topische Anwendungsformen oder Injektabilia verwendet.

Hauptsächlich ist der Einsatz am Menschen vorgesehen, der Einsatz im Veterinär-Bereich ist aber ebenso möglich.

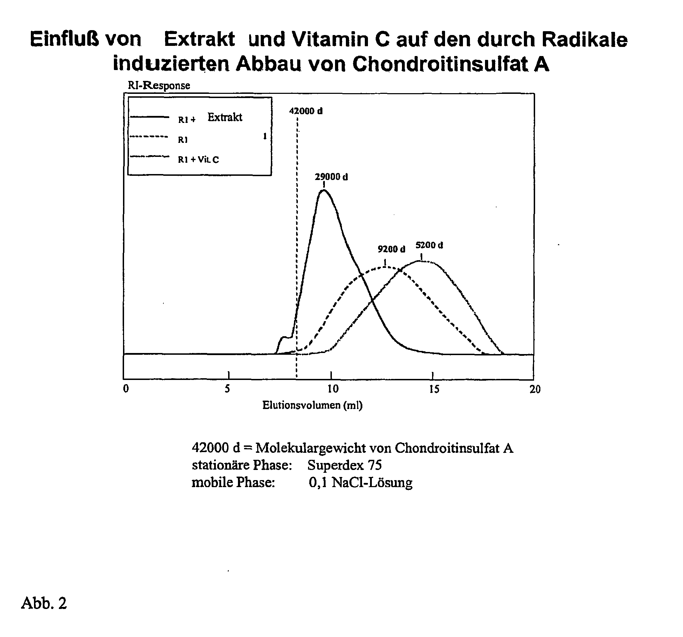

Die Aloysiaextrakte besitzen starke antioxidative Eigenschaften - wie im DPPH-Test nachgewiesen. Überraschenderweise besitzen sie jedoch keine prooxidativen Eigenschaften wie z.B. Vitamin C, die zum Abbau von Glykosaminoglykanen (z.B. Heparin, Chondroitinsulfate, Heparansulfate) führen. Die Extrakte inhibierten den Abbau von Glykosaminoglykanen, der durch freie Eisen-(II)-Ionen und Wasserstoffperoxid hervorgerufen wird (s. Abbildung 2).

Durch diese neue Wirkung der Aloysiaextrakte sind diese in der Lage den Abbau von z.B.

Knorpel bzw. den Abbau der extrazellulären Matrix, die für die Pathogenese einer Reihe von chronischen Erkrankungen essentiell ist, zu verlangsamen bzw. zu stoppen. Für die Induktion der Angiogenese ist der Abbau von Glykosaminoglykane, die Bestandteile der extrazelluären Matrix sind, von wesentlicher Bedeutung. Wirkstoffe oder Extrakte die den Abbau der extrazellulären Matrix verhindern (Matrixprotektoren) sind bis jetzt nicht bekannt.

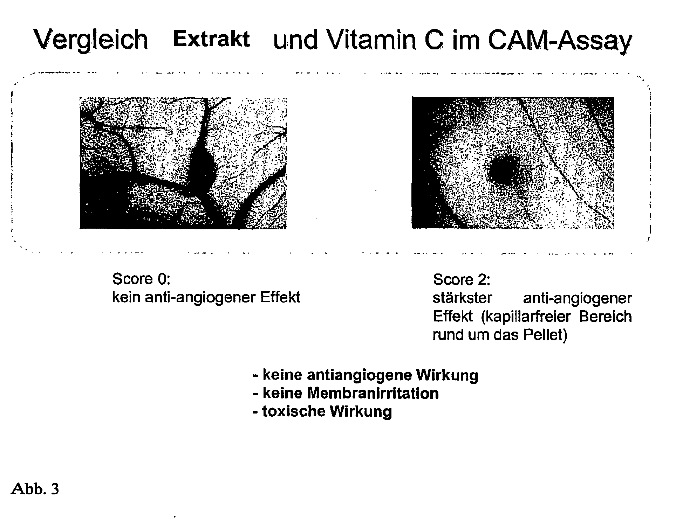

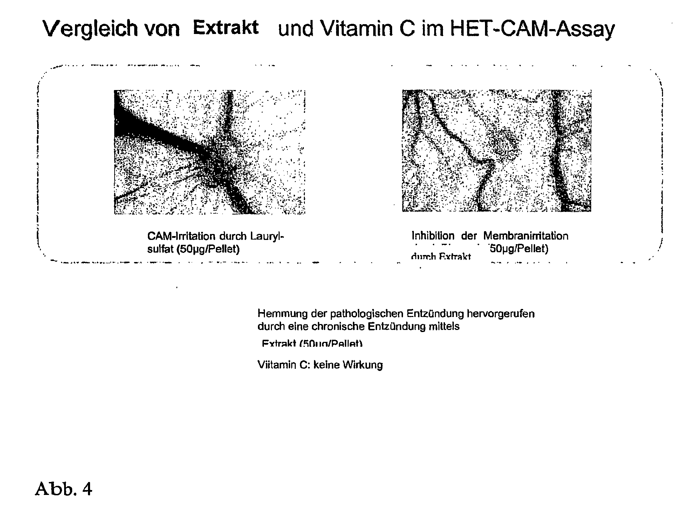

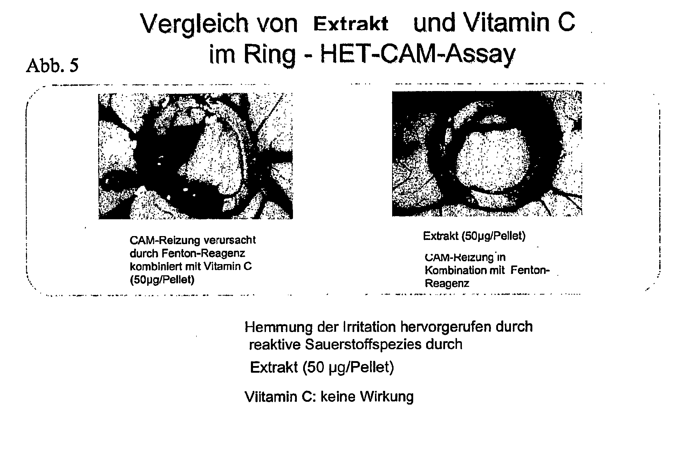

Die aus den *in vitro* Versuchen vermuteten pharmakologischen Wirkungen der beschriebenen Aloysiaextrakte sind *in vivo* durch Hemmung der pathologischen Angiogenese unterschiedlicher Genese an der Chorion-Allantois-Membran (HET-CAM) des bebrüteten Hühnereies belegt worden (s. Abbildung 4). Desweiteren wird die durch reaktive Sauerstoffspezies (ROS) hervorgerufene Irritation an der Chorio-Allantois-Membran gehemmt (s. Abbildung 5). Die physiologische Angiogenese wird durch die Aloysiaextrakte nicht gehemmt (s. Abbildung 3).

Eine intakte extrazelluläre Matrix ist wesentlich für ein gesundes Gewebe. Werden die Bestandteile abgebaut kommt es z.B. zur Knorpeldegeneration oder zur Induktion einer pathogenen Angiogenese.

Die Angiogenese ist ein physiologischer differenzierender Gewebeprozeß in der Embryonalentwicklung, nach der weiblichen Regelblutung und bei der Wundheilung. Diese Differenzierung geht von Kapillaren aus, in dem die Basalmembran stellenweise aufgelöst wird, Endothelzellen migrieren und proliferieren, sich zu einer Röhre zusammenschließen und mit benachbarten Proliferationsstellen eine Schleife bilden. Die Basalmembran wird an den neu entstandenen Gefäßen ausgebildet. Dieser Prozeß ist einer Steuerung durch antagonsierende Mediatoren unterworfen. Zu den Angiogenese-stimulierenden Faktoren zählen der Acidic Fibroblast Growth Factor (FGF-1), der Basic Fibroblast growth factor (FGF-2), der Vascular Endothelial growth Factor (VEGF), der Interleukin 1a (IL la) u.a. Endogene Inhibitoren stehen diesen stimulierenden Faktoren gegenüber und verhindern beim Gesunden die Angiogenese.

Bei verschiedenen Erkrankungen spielt in der Pathogenese die Gefäßneubildung eine Rolle. Dazu gehören vor allem Tumorerkrankungen. Sowohl das Wachstum eines soliden Tumors als auch die Metastasierung ist abhängig von der Angiogenese im Tumorgewebe. Einige weitere Beispiele für Erkrankungen, bei denen die Angiogenese eine pathogene Rolle spielt, sind oben bereits genannt.

Wirksame und nebenwirkungsarme Angiogenese-Inhibitoren stellen heute noch eine therapeutische Lücke dar, da noch keine zur Anwendung am Menschen zugelassener Angiogenese-Hemmstoff zur Verfügung steht. Es wurden zwar schon verschiedene Angiogenese-Inhibitoren, z.B. Suramin, klinisch geprüft, aber der therapeutische Nutzen wird aufgrund toxischer Wirkungen in Frage gestellt.

Mit der pharmakologischen Prüfung des Lyophilisates des Fluidextraktes von Aloysia triphylla an der CAM des Hühnerembryos trat völlig überraschend die starke Hemmung der pathogenen Angiogenese auf. Eine membranirritierende oder toxische Wirkung des Extraktes ist nicht beobachtet worden. Nebenwirkungen sind beim Gebrauch von Aloysia triphylla bisher nicht bekannt geworden.

Zur Behandlung der rheumatoiden Arthritis werden in neueren Therapiestrategien Angiogenese-Hemmstoffen gesucht, da bei der Pathogenese die synoviale Proliferation mit einer Neovaskularisierung verbunden ist. Die Unterdrückung der Proliferation von Endothelzellen kann als wichtiges Therapieziel angesehen werden, da hiermit auch eine Reduzierung des pathologisch-immunologischen Prozesses zu erwarten ist.

Die vorliegende Erfindung wird nachfolgend durch Abbildungen und Beispiele verdeutlicht. Sie ist hierauf selbstverständlich nicht beschränkt, ihr Schutzumfang wird vielmehr durch die Ansprüche bestimmt.

In den Abbildungen ist folgendes dargestellt:
- Abb. 1: zeigt einen Flow Chart für die Herstellung eines erfindungsgemäßen Extraktes;
- Abb. 2: zeigt die Hemmung des Abbaus von Glykosaminoglykanen (z.B. Chondroitinsulfat A) durch reaktive Sauerstoffspezies (ROS) durch den erfindungsgemäßen wasserlöslichen Aloysiaextrakt. Das antioxidative Vitamin C beschleunigt im Vergleich den Abbau der Glykosaminoglykanen unter physiologischen Bedingungen (pH-Wert 7,2, 37 Grad Celsius);
- Abb. 3: zeigt den Einfluß eines erfindungsgemäßen wasserlöslichen Aloysiaextrakts auf die physiologische Angiogenese im Vergleich mit Vitamin C;
- Abb. 4: stellt die Hemmung der pathologischen Angiogenese, hervorgerufen durch eine chronische Entzündung, mittels des erfindungsgemäßen wasserlöslichen Extrakts aus Aloysia im Vergleich mit Vitamin C dar;
- Abb.5: zeigt die Hemmung der durch reaktive Sauerstoffspezies (ROS) hervorgerufenen Irritation an der Chorio-Allantois-Membran.

### Beispiele:

### Beispiel 1: Herstellung des komplett wasserlöslichen Spezialextraktes aus Aloysia triphylla (s. auch Abb. 1)

Das getrocknete, geschnittene Pflanzenmaterial von Aloysia triphylla (oberirdische Bestandteile mit oder ohne Blüten, bzw. nur Blätter bzw. nur Stengel), die einen Mindestgehalt an antioxidativen Oligosacchariden enthalten, werden mit Wasser oder mit Wasser/Ethanol-Mischungen (z.B. Wasser/Ethanol = 50/50 (V:V)) bei 40 Grad Celsius über ungefähr 8 Stunden extrahiert. Das Verhältnis von getrocknetem Pflanzenmaterial zu Auszugsmittel kann variieren und beträgt 1:4 bis 1:100 Teile. Die genau verwendeten Ethanol-Wassermischungen, die Extraktionstemperatur, die Extraktionsdauer und die Menge an Auszugsmittel variieren von Charge zu Charge und sind abhängig vom Gehalt der antioxidativen Oligosaccharide und dem Gehalt an unerwünschten Begleitstoffen (wie z.B. Chlorophyll, Carotinoide und andere insbesondere lipophile Bestandteile).

Die exakten Extraktionsbedingungen werden jeweils in Vorversuchen mit Hilfe einer Analytik auf antioxidative Oligosaccharide, die, wie oben angesprochen, mit einem Mindestgehalt in den Extrakten enthalten sein sollen, ermittelt.

Nach der Extraktion wird zur Abtrennung der unlöslichen Bestandteile in einer Durchlaufzentrifuge ("Milchzentrifuge") zentrifugiert. Die abgetrennten unlöslichen Bestandteile (Trester) werden verworfen. Der Überstand (bzw. Überlauf) wird auf bis zu 4 Grad Celsius abgekühlt, jedoch mindestens auf 40 Grad Celsius. Nach einer Lagerdauer von bis zu einem Monat werden die sich abscheidenden lipophilen Komponenten (insbesondere Chlorophyll) mittels Filtration entfernt.

Anschließend wird das Filtrat bei 50 bis 90 Grad Celsius im Vakuum auf ca. ein Zehntel bis ein Viertel des Ausgangsvolumens eingeengt. Die konzentrierte Lösung wird nochmals auf bis zu 4 Grad Celsius abgekühlt, jedoch mindestens auf 40 Grad Celsius. Nach dieser Endklärung werden die sich abscheidenden bzw. ausflockenden Schwebstoffe nach einer Lagedauer von bis zu einem Monat mittels Filtration entfernt.

Das Filtrat wird anschließend mit inertem Material z.B. Maltodextrin bzw. Aerosil versetzt, um eine gewünschte Konzentration an antioxidativen Oligosacchariden im Endprodukt zu erhalten. Die Endtrocknung erfolgt im Vakuumkonzentrator oder auf dem Trockenband bei Temperaturen zwischen 40 bis 90 Grad Celsius. Das getrocknete Material wird anschließend in einer Mühle auf eine gewünschte Korngröße zerkleinert. Das Pulver wird vakuumverpackt. Das Endprodukt, das für die Herstellung von Getränken geeignet ist, löst sich in Wasser ohne Rückstand auf (insbesondere scheiden sich keine farbigen Partikel ("schwarze Pünktchen" ab). Das Endprodukt enthält einen Mindestgehalt an antioxidativen Oligosacchariden von ungefähr 10% und erfüllt die Mindestkriterien zur mikrobiologischen Reinheit nach dem lebensmittelrechtlichen Vorschriften und den Arzneibüchern. Die vorgeschriebenen Obergrenzen an Schwermetallen, Herbiziden und Pestiziden werden ebenfalls unterschritten und somit eingehalten.

### Beispiel 2: Entzündungshemmende Wirksamkeit des Extraktes von Aloysia triphylla im HET-CAM Test

Als Testpellet wurden dabei 10µl einer Lösung von 50 mg Lyophilisat (der im obigen Beispiel 1 gewonnen Zusammensetzung) in 1 ml Agaroselösung (mit 5mg Laurylsulfat/ml) auf die CAM appliziert. Im Bereich des Testpellets erschien die durch die Entzündung hervorgerufene Angiogenese gegenüber dem Kontrollversuch geringer bis hin zu einer vollständigen Unterdrückung. Dieser Versuchaufbau stellt ein anerkanntes Modell für die in vivo-Überprüfung der Hemmung der Angiogenese dar und belegt die völlig unerwarteten, neuartigen, pharmakologischen Wirkungen der genannten Lyophilisate. Siehe hierzu auch Abbildung 4.

Der HET-CAM-Test gehört zu einer Reihe von Modellversuchen, die Substanzen auf ihre Hemmung der pathogenen Angiogenese für mögliche therapeutische Anwendungen zu prüfen. Der Vorteil des HET-CAM-Testes liegt darin, dass er zu den *in vivo*-Versuchen gehört, die eine sichere Aussage über klinische Relevanz zulassen als *in vitro*-Verfahren. In diesem in vivo-Test liegt das komplexe System der Angiogenese mit all seinen Zellenfunktionen und Mediatoren vor, so dass eine vergleichsweise sichere Aussage über die Hemmwirkung der Angiogenese möglich ist. Der Test ist als Screening-Verfahren zur Ermittlung von Substanzen mit Angiogenese-hemmenden Eigenschaften anerkannt (Svahn, C.M., M. Weber, C. Mattsson, K. Neiger, M. Palm carbohydr. Polym. 18, 9-16 (1992); Hahnenberger r., A.M. Jakobsen, A. Ansari, t. Wehler, C.M. Svahn, U.Lindahl, Glycobioology 3, 567-573 (1993); und Galliardi, A., H. Hadd, D.C. Collins, Cancer Res. 52, 5073-5075 (1992)).

### Beispiel 3: Bestätigung der Ergebnisse des HET-CAM-Tests im Tierversuch

Die Ergebnisse des HET-CAM-Assays konnten außerdem in einem Tiermodell der chronischen Entzündung bestätigt werden. Die Wirkung des Extraktes wurde in einem Mäusemodell der durch Dextransulfat hervorgerufenen akuten Kolitis überprüft.

Hier wird in insgesamt 10 Mäusen eine Kolitis durch Dextransulfat induziert. Durch diese Darmentzündung verlieren die Mäuse an Gewicht. Endpunktparameter sind die Feststellung des Mausgewichts, der Darmlänge sowie die Reduktion von bekannten Interleukinen, die Entzündungen hervorrufen.

Nach Gabe von täglich 600 µg wasserlöslichem Aloysiaextrakt (über 10 Tage hinweg) war das Endgewicht von 5 Mäusen nach 10 Behandlungstagen im Vergleich zur Kontrollgruppe (5 Mäuse) signifikant erhöht (p = 0,05). Die Ergebnisse der Behandlung sind in Tabelle 1 dargestellt. Bei den angegebenen Werten handelt es sich um Mittelwerte mit den maximalen Schwankungsbereichen.

**Tabelle 1**

| Behandlung | Anfangsgewicht in Gramm (Tag 0) | Endgewicht in Gramm (Tag 10) |
|---|---|---|
| 600 µg erfindungsgemäßer Extrakt (s.o.) | 21,2 (+/- 0,8) | 19,2 (+/- 1,2) |
| PBS (Kontrolle) | 20,8 (+/- 0,5) | 15,4 (+/-1,4) |

Die IFN- und IL-10 Werte waren in den MLN-Zellen, gewonnen aus den Lymphknoten, nach Stimulation signifikant abgesenkt.

Diese Ergebnisse der Versuchs an der Maus bestätigen den erfolgreichen Einsatz der erfindungsgemäßen Zusammensetzung bei chronischen Entzündungen insbesondere des Magen-Darm-Traktes.

### Beispiel 4: Herstellung von Lebensmitteln, die den erfindungsgemäßen Extrakt enthalten

Ausgangsprodukt: Wasserlösliche Fraktion eines alkoholischen Extraktes aus Aloysia triphylla (wie oben in Beispiel 1 gewonnen), optimiert auf einen Gehalt antioxidativer Oligosaccharide von ca. 10%.)

In Tabelle 2 ist das jeweilige Mischungsverhältnis des Extraktes im Milchprodukt angegeben. Es zeigten sich die in der Tabelle aufgeführten Ergebnisse.

**Tabelle 2:**

| | Jogurt (Erdbeer) ELS 04-06/2003 | Frischkäse (Kräuter) ELS 04-06/2003 | Milch-Shake (Grundmasse) ELS 10/2003 |
|---|---|---|---|
| Dosierung | a) 1000 mg/150 g | a) 3000 mg/200 g | a) 750 mg/1 l |
| | b) 500 mg/150 g | b) 1000 mg/200 g | b) 500 mg/1 l |
| | c) 250 mg/150 g | c) 500 mg/200 g | c) 250 mg/1 l |
| Geschmack | d) bitter | d) ohne | d) ohne |
| | e) leicht bitter | e) ohne | e) ohne |
| | f) leicht bitter | f) ohne | f) ohne |
| Farbe | g) leicht bräunlich | g) leicht bräunlich | g) bräunlich |
| | h) neutral | h) neutral | h) leicht bräunlich |
| | i) neutral | i) neutral | i) neutral |
| Geruch | ohne | ohne | ohne |
| Mischbarkeit | voll mischbar | voll mischbar | voll mischbar |
| Löslichkeit | voll löslich | voll löslich | voll löslich |
| Stabilität | 90 Tage in wässrig saurer Lösung (pH4) | 90 Tage | 90 Tage |
| Haltbarkeit im Produkt | mind. 28 Tage | mind. 28 Tage | mind. 28 Tage |
| Mikrobiologie | i.O. | i.O. | i.O. |
| Scaling Up | je 1000 kg Pflaume-Müsli-Jog. Apfel-Müsli-Jogurt | 1000 kg Kräuter-Frischkäse | 3000 l Schololade-Milchshake |
| Einsatzempfehlung | in aromatisierten oder Müsli-Jogurts; in der Fruchtzubereitung | in aromatisierten oder Kräuter-Frischkäsen; in der Fruchtzubereitung | in Geschmäckern wie Schkolade, Mokka, Nuss etc. |

## Patentansprüche

1. Verfahren zur Herstellung eines Extraktes von Aloysia triphylla, das die folgenden Schritte umfasst:
a) Bereitstellen des getrockneten Krautes von Aloysia triphylla;
b) Extrahieren des Krautes mit einem wässrig-alkoholischen Auszugsmittel;
c) Zentrifugation und Abtrennen des unlöslichen Rückstandes aus dem Extrakt;
d) Filtration des Extraktes zur Abtrennung von lipophilen Komponenten;
e) Konzentrieren, Klären und Trocknen des Extraktes.

2. Verfahren nach Anspruch 1, wobei die in Schritt d) abgetrennte lipophile Komponente Chlorophyll ist.

3. Extrakt, der nach dem Verfahren nach Anspruch 1 herstellbar ist.

4. Extrakt nach Anspruch 3, wobei der Extrakt in Wasser im wesentlichen vollständig lösbar ist.

5. Extrakt nach Anspruch 3 oder 4, wobei das zur Extraktion eingesetzte Auszugsmittel Wasser und Ethanol aufweist.

6. Extrakt nach einem oder mehreren der vorhergehenden Ansprüche, wobei der Extrakt ein Fluidextrakt oder ein Spezialextrakt ist.

7. Extrakt nach einem oder mehreren der vorhergehenden Ansprüche, wobei der Extrakt antioxidative Oligosaccharide enthält.

8. Extrakt nach einem oder mehreren der vorhergehenden Ansprüche, der in lyophilisierter Form vorliegt.

9. Lebensmittel, Nutraceutical oder Kosmetikum, das einen Extrakt von Aloysia triphylla nach einem oder mehreren der Ansprüche 3-8 enthält.

10. Pharmazeutische Zusammensetzung, die einen Extrakt von Aloysia triphylla nach einem oder mehreren der Ansprüche 3-8 und einen oder mehrere pharmazeutische Hilfsstoffe/Trägerstoffe enthält.

11. Pharmazeutische Zusammensetzung nach Anspruch 10, die zur Verabreichung durch Injektion, zur systemischen und/oder topischen Verabreichung vorgesehen ist.

12. Verwendung eines Extraktes nach einem oder mehreren der Ansprüche 3-8 zur Herstellung eines Medikamentes zur Chemoprävention und Behandlung von Colitis und chronischen Erkrankungen.

13. Verwendung nach Anspruch 12, wobei die chronischen Erkrankungen rheumatoide Arthritis, und chronische Darmerkrankungen sind.

14. Verwendung eines Extraktes nach einem oder mehreren der Ansprüche 3-8 zur Herstellung eines Medikamentes zum Schutz vor dem Abbau von Knorpel.

15. Verwendung nach einem oder mehreren der Ansprüche 12-14, wobei die Dosierung der Fluidextrakt-Trockensubstanz zwischen 20mg und 2 g pro Tag liegt.

## Claims

1. A method for the preparation of an extract of Aloysia triphylla comprising the following steps:
a) providing the dried herb of Aloysia triphylla;
b) extracting the herb with an aqueous-alcoholic extractant;
c) centrifugation and separating the insoluble residue from the extract;
d) filtration of the extract to separate lipophilic components;
e) concentrating, clarifying and drying said extract.

2. The method of claim 1, wherein the component separated in step d) is chlorophyll.

3. An extract which is obtainable by the method according to claim 1.

4. The extract according to claim 3 wherein the extract is essentially completely soluble in water.

5. The extract according to claim 3 or 4 wherein the extractant employed for extraction contains water and ethanol.

6. The extract according to one or more of the preceding claims wherein the extract is a fluid extract or a specific extract.

7. The extract according to one or more of the preceding claims wherein the extract contains antioxidative oligosaccharides.

8. The extract according to one or more of the preceding claims which is present in a lyophilised form.

9. A foodstuff, nutraceutical or cosmetic containing an extract of Aloysia triphylla according to one or more of claims 3-8.

10. A pharmaceutical composition containing an extract of Aloysia triphylla according to one or more of claims 3-8 and one or more pharmaceutical adjuvants/carriers.

11. The pharmaceutical composition according to claim 10 which is designed for administration by injection, systemic and/or topic administration.

12. The use of an extract according to one or more of claims 3-8 for the preparation of a medicament for the chemoprevention and treatment of chronic diseases and colitis.

13. The use according to claim 12, wherein the chronic diseases are rheumatoid arthritis, and chronic intestinal diseases.

14. The use of an extract according to one or more of claims 3-8 for the preparation of a medicament for the protection against the degradation of cartilage.

15. The use according to one or more of claims 12-14 wherein the dosage of the fluid extract is between 20 mg and 2 g per day based on dry substance.

## Revendications

1. Procédé de production d'un extrait d'*Aloysia triphylla,* comprenant les étapes suivantes consistant à :
a) préparer l'herbe séchée d'*Aloysia triphylla* ;
b) extraire l'herbe à l'aide d'un milieu d'extraction aqueux - alcoolique ;
c) centrifuger et séparer le résidu insoluble de l'extrait ;
d) filtrer l'extrait pour séparation des composants lipophiles ;
e) concentrer, clarifier et sécher l'extrait.

2. Procédé selon la revendication 1, dans lequel le composant lipophile séparé à l'étape d) est de la chlorophylle.

3. Extrait pouvant être produit par le procédé selon la revendication 1.

4. Extrait selon la revendication 3, l'extrait étant globalement complètement soluble dans l'eau.

5. Extrait selon la revendication 3 ou 4, dans lequel le milieu d'extraction utilisé pour l'extraction contient de l'eau et de l'éthanol.

6. Extrait selon une ou plusieurs des revendications précédentes, dans lequel l'extrait est un extrait fluide ou un extrait spécial.

7. Extrait selon une ou plusieurs des revendications précédentes, dans lequel l'extrait contient des oligosaccharides anti-oxydants.

8. Extrait selon une ou plusieurs des revendications précédentes sous forme lyophilisée.

9. Aliment, produit nutraceutique ou produit cosmétique qui contient un extrait d*'Aloysia triphylla* selon une ou plusieurs des revendications 3-8.

10. Composition pharmaceutique qui contient un extrait d*'Aloysia triphylla* selon une ou plusieurs des revendications 3-8 et un ou plusieurs excipients/véhicules pharmaceutiques.

11. Composition pharmaceutique selon la revendication 10 prévue pour l'administration par injection, pour l'administration systémique et/ou topique.

12. Utilisation d'un extrait selon l'une quelconque des revendications 3-8 pour fabrication d'un médicament pour la chimioprévention et le traitement de la colite et de maladies chroniques.

13. Utilisation selon la revendication 12, dans laquelle les maladies chroniques sont la polyarthrite rhumatoïde et les maladies intestinales chroniques.

14. Utilisation selon une ou plusieurs des revendications 3-8 pour la fabrication d'un médicament pour la protection contre la dégradation du cartilage.

15. Utilisation selon une ou plusieurs des revendications 12-14, dans laquelle la dose d'extrait fluide de substance sèche est comprise entre 20 mg et 2 g par jour.
